(19) Europäisches Patentamt / European Patent Office / Office européen des brevets

(11) **EP 4 328 183 A1**

(12) **EUROPEAN PATENT APPLICATION**
published in accordance with Art. 153(4) EPC

(43) Date of publication:
**28.02.2024 Bulletin 2024/09**

(21) Application number: **22791821.6**

(22) Date of filing: **22.04.2022**

(51) International Patent Classification (IPC):
$C01B\ 39/06^{(2006.01)}$    $C01B\ 39/48^{(2006.01)}$
$C07C\ 39/08^{(2006.01)}$    $C07C\ 37/00^{(2006.01)}$
$B01J\ 37/08^{(2006.01)}$    $B01J\ 29/89^{(2006.01)}$

(52) Cooperative Patent Classification (CPC):
**B01J 29/89; B01J 37/08; C01B 39/06; C01B 39/48;
C07C 37/00; C07C 39/08**

(86) International application number:
**PCT/JP2022/018562**

(87) International publication number:
**WO 2022/225050 (27.10.2022 Gazette 2022/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.04.2021 JP 2021072560**

(71) Applicant: **Mitsui Chemicals, Inc.
Tokyo 104-0028 (JP)**

(72) Inventors:
- **HORIUCHI, Nobuhiko**
  **Sodegaura-shi, Chiba 299-0265 (JP)**
- **MATSUSHIMA, Takuma**
  **Sodegaura-shi, Chiba 299-0265 (JP)**
- **AKIYAMA, Satoshi**
  **Sodegaura-shi, Chiba 299-0265 (JP)**
- **OKABE, Akihiro**
  **Sodegaura-shi, Chiba 299-0265 (JP)**
- **IMOTO, Itsuki**
  **Sodegaura-shi, Chiba 299-0265 (JP)**
- **NAKANISHI, Keita**
  **Sodegaura-shi, Chiba 299-0265 (JP)**
- **SAITO, Susumu**
  **Sodegaura-shi, Chiba 299-0265 (JP)**
- **KUBOTA, Yoshihiro**
  **Yokohama-shi, Kanagawa 240-8501 (JP)**
- **INAGAKI, Satoshi**
  **Yokohama-shi, Kanagawa 240-8501 (JP)**

(74) Representative: **Mewburn Ellis LLP
Aurora Building
Counterslip
Bristol BS1 6BX (GB)**

(54) **METHOD FOR MANUFACTURING MODIFIED ALUMINOSILICATE, AND MODIFIED ALUMINOSILICATE AND METHOD FOR MANUFACTURING AROMATIC POLYHYDROXIDE COMPOUND USING SAME**

(57) Provided is a method for manufacturing a modified aluminosilicate in which a hydroquinone is highly selectively manufactured by a reaction between a phenol and hydrogen peroxide under industrially advantageous conditions, wherein the manufacturing method includes: a second step for treating an aluminosilicate with an acid, the highest peak (B) in a region of 17.5-35° in XRD measurement being located in a region of from 20° to less than 22.5°, and the peak intensity ratio of the peak (B) relative to the highest peak (Y) in a region of 25-27° being in a range of 1.7-5.0; a third step for performing primary calcination of the treatment product obtained in the second step; and a fourth step in which the primary calcined product obtained in the third step and liquid containing one or more elements selected from the group consisting of elements in Group 4 and Group 5 of the Periodic Table are brought into contact with each other, and drying and secondary calcination then performed.

**EP 4 328 183 A1**

**(Cont. next page)**

[Fig. 1]

XRD charts of crystals (A) of Examples and Comparative Examples

**Description**

Technical Field

The present invention relates to a method for producing a modified aluminosilicate, the modified aluminosilicate, and a method for producing an aromatic polyhydroxide compound using the same.

Background Art

Aromatic dihydroxide compounds are of importance as various organic synthesis intermediates or raw materials, and they are used as, for example, reducing agents, rubber chemicals, dyes, pharmaceuticals, agrochemicals, polymerization inhibitors, and oxidation inhibitors.

Aromatic dihydroxide compounds obtained by reacting phenols with hydrogen peroxide are, for example, hydroquinone and catechol, and production ratios of hydroquinone and catechol vary depending on production methods. In recent years, due to a demand balance between hydroquinone and catechol, a method of producing particularly hydroquinone with high selectivity has been increasingly desired.

In order to produce aromatic dihydroxide compounds by reacting phenols with hydrogen peroxide, a method for using a titanosilicate that is one of crystalline porous silicates, as a catalyst, has been disclosed (for example, Patent Literature 1 and Patent Literature 2). Moreover, Patent Literature 3 discloses a titanosilicate obtained by treating an acid-treated aluminosilicate with titanium chloride or titanium alkoxide in a gas phase.

Further, Patent Literature 4 discloses a method for producing a titanosilicate, which is obtained by mixing an aluminosilicate template material, an aluminum source, a titanium source, a silicon source, iodide, and water to prepare gel, which is then heated for crystallization and then calcinated.

The present inventors have disclosed a method for producing an aluminotitanosilicate by contacting an aluminosilicate compound with a halogenated titanium compound in liquid form. (Patent Literature 5)

Citation List

Patent Literature

[Patent Literature 1] Japanese Patent No. 4254009
[Patent Literature 2] WO2015/041137
[Patent Literature 3] JP2008-050186A
[Patent Literature 4] JP2017-057126A
[Patent Literature 5] WO2019/225549

Summary of Invention

Technical Problem

Patent Literature 5 indicates that the aluminotitanosilicate obtained in this literature can be an excellent catalyst for production of aromatic dihydroxide compounds. The aforementioned literature discloses that among them, the aluminotitanosilicate with a titanium source introduced by using titanium tetrachloride gives an aromatic dihydroxide compound with particularly high selectivity. Moreover, according to X-ray crystal structure analysis (X-ray diffraction (XRD) measurement) of the present inventors, the aluminotitanosilicate of the example in the aforementioned Patent Literature 5 was a compound exhibiting the maximum peak at 23 to 24°.

On the other hand, the aforementioned literature also discloses, that the method using a titanium trichloride aqueous solution, which is a milder method for introducing a titanium source, can also produce aromatic dihydroxide compounds with high selectivity, however, the method does not seem to be comparable to the method using titanium tetrachloride described above.

An object of the present invention is to provide a method for producing a modified aluminosilicate capable of producing hydroquinones with high selectivity by, for example, reactions of, for example, phenols with hydrogen peroxide, under more industrially advantageous conditions than those of conventional methods. Moreover, an object of the present invention is also to provide a catalyst capable of producing hydroquinones with high selectivity by, for example, reactions of, for example, phenols with hydrogen peroxide under more industrially advantageous conditions, and a method for producing aromatic polyhydroxide compounds using the same catalyst.

Solution to Problem

The present inventors have found, as a result of diligent investigations for the aforementioned problems, that when an aluminosilicate exhibiting a special diffraction pattern in XRD measurement contacts with zeolite and a silica gel to produce an aluminosilicate, even an aspect whereby a titanium source is introduced to the aluminosilicate by using a trivalent transition metal compound such as a titanium trichloride aqueous solution can be a modified aluminosilicate like aluminotitanosilicate capable of producing hydroquinones with extremely high selectivity, and that in an aspect using a tetravalent transition metal compound such as titanium tetrachloride, a modified aluminosilicate like alumino(titano)silicate with even higher performance can be obtained, and thus have completed the present invention.

Namely, the present invention includes the items described in [1] to [8].

[1] A method for producing a modified aluminosilicate, comprising

a first step of contacting gel-like silica, zeolite, and crystals (A) that is an aluminosilicate with the highest peak ($\alpha$) at 22.5 to 25.0° in a region of 17.5 to 35° as measured by XRD, to obtain an aluminosilicate (A-1'),
a second step of treating the aluminosilicate (A-1') obtained in the first step with an acid,
a third step of subjecting the treated product obtained in the second step to primary calcination at 550°C to 850°C, and
a fourth step of contacting the calcined product obtained in the third step and liquid containing one or more elements selected from the group consisting of elements in Group 4 and Group 5 of the Periodic Table, followed by carrying out drying and secondary calcination.

[2] The method for producing a modified aluminosilicate according to [1], wherein the crystals (A) in the first step are obtained by a process including a step of contacting gel-like silica with zeolite and treating the same at 155 to 168°C for 30 to 40 hours.

[3] An aluminosilicate (A-1), having the highest peak ($\beta$) in a region of 20° or more and less than 22.5° in a region of 17.5 to 35° as measured by XRD, wherein a peak intensity ratio of the peak ($\beta$) to the highest peak ($\gamma$) in a region of 25 to 27° is in a range of 1.7 to 5.0.

[4] The aluminosilicate (A-1) according to [3], wherein the peak intensity ratio is in a range of 1.7 to 4.0.

[5] A catalyst for production of aromatic polyhydroxide compounds, comprising the modified aluminosilicate according to [1].

[6] A method for producing a modified aluminosilicate, comprising

a second step of treating the aluminosilicate (A-1) according to [3] with an acid,
a third step of subjecting the treated product obtained in the second step to primary calcination at 550°C to 850°C, and
a fourth step of contacting the calcined product obtained in the third step and liquid containing one or more elements selected from the group consisting of elements in Group 4 and Group 5 of the Periodic Table, followed by carrying out drying and secondary calcination.

[7] A catalyst for production of aromatic polyhydroxide compounds, comprising the modified aluminosilicate according to [6].

[8] A method for producing an aromatic polyhydroxide compound, comprising a step of reacting an aromatic hydroxide with hydroperoxide in the presence of the catalyst according to [5] or [7].

Advantageous Effects of Invention

According to the method for producing the modified aluminosilicate described above, one of its characteristics is that an industrially useful modified aluminosilicate is obtained from an aluminosilicate having a specific crystal structure as a raw material. Even when such a modified aluminosilicate is brought into contact, under mild conditions, with liquid containing one or more elements selected from the group consisting of elements in Group 4 and Group 5 of the Periodic Table, such as an aqueous solution of a titanium source, it can obtain an aluminotitanosilicate used for catalysts suitable for production of aromatic polyhydroxide compounds. For this reason, the present invention is of industrial importance. Further, use of the modified aluminosilicate produced by the aforementioned production method enables production of aromatic dihydroxide compounds such as hydroquinone, for example, by reacting phenols with hydrogen peroxide.

Brief Description of Drawings

[Fig. 1] Fig. 1 is a chart illustrating the results of X-ray diffraction measurements of the aluminosilicate crystals (A) used in Examples and Comparative Examples.
[Fig. 2] Fig. 2 is a chart illustrating the results of X-ray diffraction measurements of the aluminosilicates obtained in Examples and Comparative Examples.
[Fig. 3] Fig. 3 is a chart illustrating the results of X-ray diffraction measurements of the aluminosilicate obtained in Examples and Comparative Examples.
[Fig. 4] Fig. 4 is a chart illustrating the results of X-ray diffraction measurements of the aluminosilicate obtained in Examples and Comparative Examples.

Description of Embodiments

Embodiments according to the present invention will be described in detail below.

<Production method of modified aluminosilicate by using aluminosilicate (A-1')>

The method for producing a modified aluminosilicate of the present invention includes a first step of contacting gel-like silica, zeolite,

and crystals (A) that is an aluminosilicate with the highest peak ($\alpha$) at 22.5 to 25.0° in a region of 17.5 to 35° as measured by XRD (hereinafter also simply referred to as "crystals (A)"), to obtain an aluminosilicate (A-1'),
a second step of contacting the aluminosilicate (A-1') obtained in the first step with an acid,
a third step of subjecting the treated product obtained in the second step to primary calcination at 550°C to 850°C, and
a fourth step of contacting the calcined product obtained in the third step and liquid containing one or more elements selected from the group consisting of elements in Group 4 and Group 5 of the Periodic Table, followed by carrying out drying and secondary calcination.

[Step 1]

The first step is a step of producing the aluminosilicate (A-1') by contacting the crystals (A) with gel-like silica as a silicon source and zeolite as an aluminum source.

First, the crystals (A) used in the first step will be described. Here, the crystals (A) preferably contains no elements in Group 4 and Group 5, or contains them to the extent that they do not affect the present invention, even though contained. Examples of elements in Group 4 include, for example, titanium, zirconium, and hafnium. Examples of elements in Group 5 include, for example, vanadium. Among these elements, the elements in Group 4 are preferred, and titanium, zirconium, and hafnium are more preferred, and titanium is further preferred. The aforementioned elements may be used singly or in combinations of two or more thereof.

The crystals (A) and aluminosilicate are preferably porous. Hereinafter, the crystalline and porous aluminosilicate is simply referred to as a crystalline porous aluminosilicate.

The crystals (A) has a structure with the highest peak ($\alpha$) at 22.5 to 25.0° in a region of 17.5 to 35° in XRD measurement. The lower limit value of the range of angles exhibiting the highest peak ($\alpha$) is preferably 22.7°, more preferably 23.0°, and further preferably 23.3°. On the other hand, the upper limit value thereof is, preferably 24.7° and more preferably 24.5°.

The crystals (A) having the structure identified by such XRD measurement can be considered as a so-called "seed crystals" when producing the aluminosilicate as described below. By using the crystals (A) having the structure described above, an aluminosilicate can be obtained as a raw material for catalysts suitable for production of aromatic polyhydroxide compounds as described below.

The crystals (A) are preferably porous crystalline bodies having at least a portion in which a $SiO_4$ tetrahedron with silicon at the center, arranged with oxygen at the four vertices, and an $AlO_4$ tetrahedron, arranged with aluminum in place of silicon at the center, are regularly bonded in three dimensional manner.

The crystals (A) are not particularly limited as long as they have the aforementioned structure, however, they are preferably crystalline porous aluminosilicates with an MSE type structure (hereinafter referred to as "MSE framework") in the structure code of the International Zeolite Association and are particularly preferably with, for example, UZM-35, MCM-68, and YNU-3.

The crystalline porous aluminosilicate with the aforementioned MSE framework has a three-dimensional porous structure with a 10-membered ring structure composed of 10 units of the tetrahedrons and a 12-membered ring structure composed of 12 units of the tetrahedrons.

Methods for obtaining the crystals (A) can include a method for contacting a publicly known silicon source such as publicly known colloidal silica, an aluminum source such as zeolite, and preferably an organic structure-directing agent such as dimethyldipropylammonium hydroxide or a publicly known alkali source, then stirring and heating them. The aforementioned colloidal silica can also be used in a sol condition by preliminarily heating it to an elevated temperature such as 50°C to 100°C, which is also a preferred method. In this case, a temperature range of the heating is preferably 155 to 168°C and this temperature is preferably maintained for 30 to 40 hours. The lower limit value of the temperature is more preferably 157°C, and further preferably 158°C. On the other hand, the upper limit value is, more preferably 167°C and further preferably 166°C.

Setting such a temperature range tends to facilitate production of aluminosilicates with a crystal structure that diffracts strongly at the specific angle described above. Note, however, in a stage of producing the crystals (A), a temperature is not set at a temperature substantially higher than 168°C. (the term "substantially" means that for example, when controlling temperature, in the heating step described above, a temperature exceeding 168°C for a few minutes is acceptable.).

Next, the aluminosilicate (A-1') will be described. The aluminosilicate (A-1') obtained in the first step of the present invention can be, in principle, produced by the same method as in production of the crystals (A). However, it is essential to contact gel-like silica as the silicon source and zeolite as the aluminum source in the presence of the crystals (A). Moreover, temperature and time of heating in the first step are arbitrary as long as they do not contradict the object of the invention. The lower limit of temperature is preferably 130°C and more preferably 150°C. On the other hand, the upper limit value is, preferably 700°C.

Further, the lower limit of time is preferably 10 hours and more preferably 20 hours. On the other hand, the upper limit value thereof is, 100 hours and more preferably 90 hours.

Use of the crystals (A) as so-called seed crystals promotes crystal growth upon synthesis of the aluminosilicate (A-1'). The amount of crystals (A) used is preferably 1 to 40% by weight of silica that is the silicon source, and more preferably 2 to 30% by weight. Zeolite that is the aluminum source, also serves as the silicon source, and its amount used is preferably 5 to 50% by weight of silica and more preferably 8 to 40% by weight.

The aluminosilicate (A-1') thus obtained preferably has the highest peak (β) in the region of 20° or more and less than 22.5° in the region of 17.5 to 35° as measured by XRD, and a peak intensity ratio of the peak (β) to the highest peak (γ) in the region of 25 to 27° is 1.7 to 5.0. The lower limit value of the peak intensity ratio is preferably 1.72, more preferably 1.8, and particularly preferably 1.9. On the other hand, the upper limit value is, 4.5, more preferably 4.2, further preferably 4.0, and particularly preferably 3.5. Among these, the upper limit value is further preferably 3.0 and particularly preferably 2.5.

Using such aluminosilicates (A-1') makes it possible to obtain, for example, a catalyst suitable for aromatic polyhydroxide compounds by using the method for introducing, for example, a titanium source, as described below, although the reason thereof is not known at this stage. Provided that the aluminosilicate used in the second step described below has the highest peak (β) in the region of 20° or more and less than 22.5° in the region of 17.5 to 35° as measured by XRD, and that a peak intensity ratio of the peak (β) to the highest peak (γ) in the region of 25 to 27° is in the range of 1.7 to 5.0 described above, preferably in a range of 1.7 to 4.5, and more preferably in a range of 1.7 to 4.0, a catalyst for aromatic polyhydroxide compounds having the characteristics described below can be obtained, even if the aluminosilicate (A-1') obtained by the first step as described above is not used. In the present invention, the any aluminosilicate having the specific requirements of "having the highest peak (β) in the region of 20° or more and less than 22.5° in the region of 17.5 to 35° as measured by XRD, wherein a peak intensity ratio with respect to the highest peak (γ) in the region of 25 to 27° is in the range of 1.7 to 5.0" is defined as an aluminosilicate (A-1), and the details thereof will be described below.

The aforementioned aluminosilicate (A-1') exhibits a crystal structure; however, its crystal form is probably different from those of conventional aluminosilicates (in particular different in terms of a value of the intensity ratio of the above peak (β) to peak (γ)), which is presumed to have a somewhat unstable structure. Such instability is presumed to lead to excellent performance as a catalyst for production of aromatic polyhydroxide compounds in a case in which the aluminosilicate (A-1') is converted to the modified aluminosilicate described below.

Note that, when the element of the present invention selected from elements in Group 4 and Group 5 of the Periodic Table described below is introduced into a calcined product of the aluminosilicate (A-1') obtained through the second step and third step described below, a site where the aforementioned element is introduced can be considered to be mainly on a solid surface of the calcined product of the aluminosilicate (A-1'), and therefore its crystal structure before and after the introduction of the above elements can be considered to be almost the same.

Examples of raw materials for the gel-like silica include, for example, colloidal silica, sodium silicate, wet silica, and dry silica. These silicon sources can be used singly or in combinations of two or more thereof.

The aforementioned aluminum sources may be combined for use with components other than zeolite. For example, water-soluble aluminum compounds can be used. Examples of the water-soluble aluminum compounds include, for example, aluminum hydroxide, sodium aluminate, aluminum nitrate, and aluminum sulfate. These aluminum sources can be used singly or in combinations of two or more thereof.

The alkali source described above that is, for example, hydroxides containing alkali metals can be used. Examples of hydroxides containing alkali metals include, for example, sodium hydroxide, and potassium hydroxide. These alkali sources can be used singly or in combinations of two or more thereof.

As the organic structure-directing agents described above, publicly known compounds such as N,N,N',N'-tetraethylbicyclo[2,2,2]oct-7-ene-2,3:5,6-dipyrrolidinium diiodide and N,N,N',N'-tetraethylbicyclo[2,2,2]oct-7-ene-2,3:5,6-dipyrrolidinium diiodide, can be used.

The aluminosilicate (A-1') is preferably calcined before the second step described below. Methods of the aforementioned calcination are not particularly limited, and include a method of calcination by using, for example, an electric furnace, or a gas furnace. Calcination conditions are preferably those of heating for 0.1 to 20 hours in an atmospheric atmosphere. A calcination temperature is preferably from 550°C to 850°C and more preferably 600°C to 800°C.

[Second Step]

A step of treating the aluminosilicate (A-1') obtained in the first step with an acid, in other words, a step of contacting the aluminosilicate (A-1') with an acid, is the second step.

Examples of the acids used in this step include, for example, inorganic acids, organic acids, and mixtures thereof, and specific examples thereof include, for example, nitric acid, hydrochloric acid, sulfuric acid, citric acid, oxalic acid, and mixtures thereof. Among these, an acid containing an element selected from the group consisting of elements in Group 15 and Group 16 of the Periodic Table is preferred and nitric acid is particularly preferred. A concentration of the acid is not particularly limited, but it is preferably 5% to 80% by weight and more preferably 40% to 80% by weight. When the acid is used as an aqueous solution, the amount thereof used is preferably from 1 to 100 parts by weight per 1 part by weight of the aluminosilicate (A-1'). The lower limit value thereof is more preferably 2 parts by weight, further preferably 3 parts by weight, and particularly preferably 5 parts by weight. Further preferred lower limit value is 10 parts by weight and more preferably 20 parts by weight. On the other hand, the upper limit value is, more preferably 70 parts by weight and further preferably 50 parts by weight.

A temperature condition for contacting the aforementioned aluminosilicate (A-1') with an acid is preferably 50°C to 170°C and more preferably 130°C to 170°C. A time of treatment with the acid is preferably 5 to 48 hours and more preferably 12 to 36 hours. The lower limit thereof is further preferably 18 hours. The contact with this acid is considered to partially remove aluminum from the aluminosilicate. Mainly aluminum on the surface of the aluminosilicate is presumed to be removed. It is assumed that selecting relatively high temperature and prolonged time conditions as described above facilitate forming of a structure favorable for introduction of elements in Group 4 and Group 5 described above in the calcination treatment in the third step described below.

[Third step]

In the third step, the treated product obtained in the above second step, i.e., the aluminosilicate (A-1') contacted with an acid, undergoes primary calcination. Calcination methods are not particularly limited, and include a method of using, for example, an electric furnace or a gas furnace. Calcination conditions are preferably those of heating for 0.1 to 20 hours in an atmospheric atmosphere. A calcination temperature is from 550°C to 850°C and more preferably from 600°C to 800°C. The primary calcination under this relatively high temperature environment is presumed to provide a favorable environment for formation of the so-called modified aluminosilicate obtained in the fourth step, which contains elements in Group 4 and Group 5 of the Periodic Table, with titanium species as representative examples, in a highly active condition.

Preferably before calcination of the aluminosilicate (A-1') contacted with the above acid, the treated product is filtered by using, for example, a nutsche to separate, for example, the acid (aqueous solution) used, and a solid portion (filtrate) is then washed and dried. The washing step described above is preferably carried out while maintaining a wet condition without drying before washing. Methods of drying after washing are not particularly limited, but those of uniform and rapid drying are preferred, and for example, external heating methods such as hot air drying and superheated steam drying, and electromagnetic wave heating methods such as microwave heating and drying and high frequency dielectric heating and drying, can be employed.

[Fourth step]

In the fourth step, the calcined product obtained in the above third step (hereinafter also referred to as "primary calcined product") is brought into contact with liquid containing one or more elements selected from the group consisting of elements in Group 4 and Group 5 of the Periodic Table as an element source in a liquid phase condition, followed by drying and subjected to secondary calcination. Examples of the elements in Group 4 and Group 5 of the Periodic Table can include, for example, titanium, zirconium, hafnium, and vanadium. The elements are preferably titanium, zirconium, and vanadium, with titanium being particularly preferred. Examples of compounds containing these elements can include halides, alkoxides and salts of inorganic acids, of each element as suitable compounds. More preferably, the halides are chlorides, and alkoxides are forms containing alkoxy groups having 1 to 6 carbon atoms. Examples of the alkoxides can further preferably include ethoxides, butoxides (n-butoxide, i-butoxide, s-butoxide, t-butoxide), and sulfates. Two or more of these compounds may be, of course, combined for use.

An aspect in which liquid containing titanium (titanium source in liquid phase), which is the most preferred aspect among elements in Group 4 and Group 5 of the Periodic Table, is used, will be described below as a representative example.

The titanium source in liquid phase is liquid containing titanium. Examples of the liquid containing titanium include, for example, a titanium compound in liquid form itself or an aqueous solution of a titanium compound. Among these, a titanium compound that is substantially acidic in its liquid condition is a preferred aspect.

Examples of titanium compounds in liquid form include, for example, titanium tetrachloride ($TiCl_4$), and titanium tetrabutoxide, with titanium tetrachloride being preferred among them. Examples of aqueous solutions of titanium compounds include, for example, a titanium tetrachloride aqueous solution, titanium trichloride ($TiCl_3$) aqueous solution, a titanium sulfate ($Ti(SO_4)_2$) aqueous solution, and a potassium hexafluorotitanate aqueous solution, with the titanium tetrachloride aqueous solution, titanium trichloride aqueous solution, and titanium sulfate aqueous solution being preferred among them. In the present invention, in addition to titanium tetrachloride, titanium sources with lower reactivity than titanium tetrachloride, such as titanium trichloride and titanium sulfate, can also be used to exhibit the catalytic activity described below. In particular, they tend to enable achievement of catalytic activity providing aromatic polyhydroxide compounds with high selectivity.

The liquid containing titanium described above can be used singly or in combinations of two or more types thereof. The above titanium-containing liquids can also be commercially available or can also be prepared by appropriately diluting a solid titanium compound with water to a desired concentration. Compared to a titanium source in gas phase, the titanium source in liquid phase (titanium-containing liquid) is less likely to leak and improves a corrosion problem on, for example, production machinery and analysis equipment, thereby facilitating implementation of industrial manufacturing.

Conditions under which the above primary calcined product is brought into contact with a titanium source are not particularly limited, but this contact introduces titanium into the primary calcined product of the aluminosilicate, and it is considered that this introduction of titanium introduces titanium in the position of aluminum that is thought to have been removed in the second step above, and causes a reaction in which aluminum in the aluminosilicate (A-1') is partially replaced by titanium. As specific conditions for this step, for example, when using a titanium compound in liquid form itself, the titanium compound in liquid form is preferably added in an amount of 5 to 300 parts by weight per 1 part by weight of the primary calcined product and more preferably in an amount of 20 to 250 parts by weight. When using an aqueous solution of a titanium compound, the aqueous solution of a titanium compound is preferably added in an amount of 1 to 10 parts by weight per 1 part by weight of the primary calcined product and more preferably in an amount of 1 to 7 parts by weight. A concentration of the aqueous solution varies depending on compounds used, but it is, for example, 10 to 70% by weight and preferably 15 to 60% by weight.

As for the amount of titanium compound in the aqueous solution, the lower limit value thereof is preferably 0.1 g per 1 g of the primary calcined product, more preferably 0.2 g, further preferably 0.3 g, and particularly preferably 0.5 g. On the other hand, the upper limit value thereof is, preferably 10 g, more preferably 5 g, and further preferably 3 g.

The above titanium source may contact the primarily calcined aluminosilicate (A-1') only once or each component may be separated plural times and used, as long as a ratio of the weights used is within the range of the above amounts added. For example, a titanium source may be added to the primary calcined product, and the calcined product obtained by carrying out drying and secondary calcination as described below may undergo drying and secondary calcination by adding a titanium source again. When adding the titanium source above, it is preferably added under a nitrogen atmosphere because hydrogen chloride is generated by a reaction between moisture in air and the titanium compound.

More specifically preferred methods can include a method of contacting the primary calcined product with the titanium source and thoroughly mixing them, followed by heat treatment of the mixture, and a method for carrying out secondary calcination after sufficient drying them by a method similar to the drying method exemplified in the third step above. Temperatures in the aforementioned heat treatment or drying treatment are not particularly limited, but they are preferably, for example, in a temperature range of 20 to 150°C in order to effectively introduce titanium into the primary calcined product. The lower limit value is more preferably 30°C, further preferably 40°C, and particularly preferably 50°C. On the other hand, the upper limit value is, more preferably 140°C, further preferably 120°C, and particularly preferably 100°C. A time required for the above step is also not particularly limited, but it is preferably from 0.1 to 24 hours. The lower limit value is more preferably 0.3 hours, further preferably 0.4 hours, and particularly preferably 0.5 hours. On the other hand, the upper limit value is, more preferably 12 hours and further preferably 6 hours. Methods of secondary calcination are not particularly limited, and, for example, an electric furnace or a gas furnace, can be used for calcination. The calcination conditions are preferably between 400°C and 850°C for 0.1 to 20 hours in an atmospheric atmosphere. The lower limit value of the calcination temperature is more preferably 500°C, further preferably 550°C, and particularly preferably 600°C. On the other hand, the upper limit value is, more preferably 800°C, further preferably 750°C, and particularly preferably 700°C.

Temperatures upon the primary calcination and secondary calcination, described above can be independently selected. Preferably it is preferable when the temperature upon secondary calcination be higher than the temperature upon the primary calcination.

By the fourth step, a crystalline porous aluminotitanosilicate that is a suitable aspect of the modified aluminosilicate of the present invention, in which, for example, aluminum in the crystalline porous aluminosilicate is thought to be partially replaced by titanium, can be obtained.

Before the above drying treatment, a mixture of the titanium source and the primary calcined product may be heated to preliminarily remove moisture and, for example, filtered to remove foreign substances, washed with an organic solvent, and then dried and undergoes secondary calcination.

The above production conditions can be applied to the case where elements other than titanium are used.

Examples of compounds that contain elements in Group 4 of the Periodic Table and can be used in place of the above titanium sources include, for example, zirconium tetrachloride, tetraalkoxy zirconium, hafnium tetrachloride, tetraalkoxy hafnium, and zirconium sulfate, and they can be liquefied for use combined with, for example, water, an alcohol, or an ether as necessary. For example, aqueous solutions of those compounds and solution of, for example, alcohols or ethers, are included. Examples of elements in Group 5 of the Periodic Table which can be used in place of the aforementioned titanium sources include, for example, vanadium pentachloride, vanadium sulfate, vanadyl trichloride, and alkoxy-substitutes thereof, and they can also be liquefied for use combined with, for example, water, alcohols, or ethers as necessary. For example, aqueous solutions of those compounds and solution of, for example, alcohols or ethers, can be included.

[Modified aluminosilicate]

The modified aluminosilicate obtained in the fourth step, as the aluminosilicate (A-1') obtained in the first step, is preferably a crystalline porous aluminosilicate having crystallinity and porosity, and more preferably a crystalline porous aluminosilicate having an MSE framework, further preferably a crystalline porous aluminosilicate with UZM-35, MCM-68, or YNU-3 structures, and particularly preferably a crystalline porous aluminotitanosilicate. The term of crystallinity can be regarded as the same aspect as that described for the crystals (A).

A crystalline porous aluminosilicate is a porous crystalline body having at least a portion in which a $SiO_4$ tetrahedron with silicon at the center, arranged with oxygen at the four vertices, and an $AlO_4$ tetrahedron arranged with aluminum in place of silicon at the center, are regularly bonded in three dimensional manner and can be said to be one type of zeolite that typically contains an aluminosilicate. A modified aluminosilicate with crystallinity and porosity contains, for example, aluminum and one or more elements selected from the group consisting of elements in Group 4 and Group 5 of the Periodic Table in the above crystalline porous aluminosilicate framework, and preferably it is obtained in such a method for replacing a portion of the aluminum in the crystalline porous aluminosilicate framework by one or more elements selected from the group consisting of elements in Group 4 and Group 5 of the Periodic Table. As a more suitable example, when one or more elements selected from the group consisting of elements in Group 4 and Group 5 of the Periodic Table are titanium, the crystalline porous modified aluminosilicate is a crystalline porous aluminotitanosilicate and the crystalline porous aluminotitanosilicate contains aluminum and titanium in its aluminosilicate framework, and preferably it is obtained by such a method for replacing a portion of the aluminum in its framework by titanium.

The crystalline porous aluminosilicate with the above MSE framework has a three-dimensional pore structure with a 10-membered ring structure composed of 10 units of the above tetrahedrons and a 12-membered ring structure composed of 12 units of the above tetrahedrons. A pore with the 12-membered ring structure is considered to facilitate diffusion of a substrate into an inside of the pore, and therefore high catalytic activity is considered to be likely obtained. Moreover, the absence of a large cavity inside the pore is considered to be one of factors that facilitates selectivity at the para position in an oxidation reaction of phenol.

It is well known that porous compounds have a wide specific surface area. The modified aluminosilicate of the present invention preferably has a specific surface area of 50 to 1000 $m^2/g$. The lower limit value of its specific surface area is more preferably 100 $m^2/g$ and further preferably 150 $m^2/g$. On the other hand, the upper limit value of its specific surface area is, preferably 800 $m^2/g$, and even more preferably 600 $m^2/g$.

The above specific surface area value can be determined by a publicly known calculation method based on BET theory by creating a BET plot from measurement results, by using a publicly known nitrogen adsorption/desorption measurement apparatus method (for example, a BELSORP-max manufactured by MicrotracBEL, Corp.).

A preferred pore volume range of the modified aluminosilicate of the present invention is 0.1 to 0.5 cm$^3$/g and more preferably 0.2 to 0.4 cm$^3$/g.

The content of each element in Group 4 and Group 5 in the modified aluminosilicate of the present invention is not particularly limited. For example, when titanium is contained as one or more elements selected from the group consisting of elements in Group 4 and Group 5 in the above modified aluminosilicate, a molar ratio of silicon to titanium ([Si]/[Ti]) is preferably in a range of 0.1 to 100, more preferably in a range of 0.5 to 50, and further preferably in a range of 1 to 30, and most preferably in a range of 2 to 30.

When a compound such as $TiCl_3$, which facilitates crystallization of itself, is used, the element may be introduced into an aluminosilicate surface in cluster form or crystal form. In this case, the [Si]/[Ti] ratio tends to be small because the apparent element content may increase. The range of the [Si]/[Ti] ratio in such a case is preferably 0.5 to 30. The lower limit value thereof is more preferably 1 and further preferably 1.2. On the other hand, the upper limit value is, more preferably 20 and further preferably 15.

The aluminum content of modified aluminosilicate of the present invention is not particularly limited, but it is preferably as a molar ratio of silicon to aluminum ([Si]/[Al]) in a range of 5 to 100,000, more preferably in a range of 10 to 10,000, and most preferably in a range of 100 to 1,000.

The modified aluminosilicate of the present invention is preferably characterized by absorption in a specific wavelength region in UV-visible absorption spectrum measurements.

The modified aluminosilicate according to the present invention preferably contains an element selected from the group consisting of elements in Group 4 and Group 5 of the Periodic Table and exhibits an absorbance at 300 nm (A[300]) in a UV-visible spectrum, which is usually 1.0 or more. Specific examples and suitable examples, for example, of the elements selected from the group consisting of elements in Group 4 and Group 5 contained in the modified aluminosilicate of the present invention are the same as those described in the "Fourth step" section above.

Moreover, when an element selected from the group consisting of elements in Group 4 and Group 5 of the Periodic Table is contained in the modified aluminosilicate in a tetravalent or pentavalent form, or when a tetravalent or pentavalent compound of the element selected from the group consisting of elements in Group 4 and Group 5 of the Periodic Table is used in the aforementioned fourth step, the A[300] is preferably greater than 0.20.

When the A[300] is less than 1.0 or, in the aforementioned specific case, less than 0.20, selectivity of aromatic dihydroxide compounds tends to be relatively low upon producing the aromatic dihydroxide compounds by a reaction of phenols and hydrogen peroxide. Incidentally, methods used to measure an absorbance are not particularly limited, and it may be the results measured by either a transmission method or reflection method. In the case of measurement by the reflection method, reflected light may include diffuse reflected light as well as normal reflected light, but for the sake of convenience, calculation is carried out based on the assumption that all light is normal reflected light.

The detailed mechanism thereof is unknown, however, the present inventors conjecture as follows (hereinafter, as the modified aluminosilicate, an example of a crystalline porous aluminotitanosilicate in which the element selected from the group consisting of elements in Group 4 and Group 5 is titanium, as described above, will be described.).

Aluminum and titanium are presumed to be present on a framework surface portion of the crystalline porous aluminotitanosilicate. They are also presumed to be preferably present mainly in an inside of concave portions such as pores of the crystalline porous aluminotitanosilicate. Here, for example, in the case of the aspect that is produced by the method for using gaseous titanium tetrachloride at elevated temperatures as described in Patent Literature 3, and is considered to contain only titanium fully incorporated without defects as a framework of a crystal structure, an absorbance in the vicinity of 300 nm in a UV-visible spectrum is presumed to be unlikely to be 1.0 or higher, or higher than 0.2 in the aforementioned specific case. In other words, the crystalline porous aluminotitanosilicate that meets the requirements of the present invention is presumed to have titanium in large amount that is incompletely incorporated into its basic framework structure with an unstable structure. It is presumed that such titanium species significantly contribute to a formation reaction of aromatic dihydroxide compounds, and when an oxidation reaction of phenols with hydrogen peroxide proceeds, a 1,2-hydroxide compound (for example, catechol), which is considered to be sterically disadvantageous due to its instability, and benzoquinone in a form where the reaction further proceeds are inhibited from being formed, which enables production of a 1,4-type aromatic dihydroxide compound with high selectivity.

From the viewpoint of higher selectivity of an aromatic dihydroxide compound, the lower limit value of A[300] of the modified aluminosilicate is preferably 1.5 and more preferably 1.8. Moreover, in the case where the element selected from the group consisting of elements in Group 4 and Group 5 of the Periodic Table described above is tetravalent or pentavalent, the lower limit value of A[300] is more preferably 0.25 and further preferably 0.30. On the other hand, there is no essential significance in setting the upper limit value of the A[300], but the upper limit value thereof is preferably 15 and more preferably 10. Moreover, the A[300] is 0.20 or less for common crystalline porous aluminotitanosilicates.

A ratio of the A[300] to an absorbance at 210 nm of a UV-visible spectrum (A[210]), (A[300]/A[210]), of the modified aluminosilicate is preferably 0.5 or more, more preferably 0.6 or more, and particularly preferably 0.8 or more. In a case in which the element selected from the group consisting of elements in Group 4 and Group 5 of the Periodic Table described above is contained in an aluminosilicate as a tetravalent or pentavalent element, or in a case in which a tetravalent or pentavalent compound is used in the fourth step described above, the "A[300]/A[210]" is preferably a value exceeding 0.10. The lower limit value thereof is more preferably 0.11. For example, when titanium that has been incorporated without defects in a framework with a crystal structure, is contained, the A[210] is relatively higher compared to the A[300], and therefore the A[300]/A[210] is usually 0.10 or less for common crystalline porous aluminotitanosilicates. There is no significant meaning in the upper limit value of the A[300]/A[210], but it is more preferably 1.5 and further preferably 1.0.

Note, however, the aforementioned UV-visible spectra can be measured by ordinary methods. For example, the following method can be included: "A method for placing 0.1 g of an aluminosilicate sample in solid state in a cell with an optical path length of 10 mm and measuring it in a wavelength range of 200 to 800 nm by using a UV-2550 type UV-visible analyzer manufactured by Shimadzu Corporation." Needless to say, the aforementioned aluminosilicate sample in solid state is preferably sufficiently dried before use.

<Aluminosilicate (A-1)>

One embodiment of the present invention is an aluminosilicate (A-1) having the highest peak ($\beta$) in the region of 20° or more and less than 22.5° in the region of 17.5 to 35° as measured by XRD, wherein a peak intensity ratio of the peak ($\beta$) to the highest peak ($\gamma$) in the region of 25 to 27° is in the range of 1.7 to 5.0. The preferred lower limit value of the peak intensity ratio is 1.72 and it is more preferably 1.8 and particularly preferably 1.9. On the other hand, the preferred upper limit value thereof is, 4.5, and it is more preferably 4.2, further preferably 4.0, and particularly preferably 3.5, among which 3.0 is further preferred and 2.5 is particularly preferred.

The aluminosilicate (A-1) is a novel substance and can be considered to be crystals with a somewhat unstable structure, which meets the requirements for peak intensity in the XRD measurements described above. As described above, such characteristics may lead to performance as the catalyst for production of aromatic polyhydroxide compounds as described above.

The method for producing an aluminosilicate (A-1') described in the first step can be considered as one aspect of the methods for producing an aluminosilicate (A-1).

As another method for producing the aluminosilicate (A-1), for example, it can also be produced by changing other specific conditions of the first step described above, even in the case of using the aluminosilicate crystals (B) other than the crystals (A), as described above, as seed crystals. Examples of methods using such crystals other than the crystals (A) can include, for example, a method for using sol-like silica containing water instead of the aforementioned gel-like silica, and controlling a molar ratio of water to silica to a specific range (Z method), as one example. Another method is to use specific gel-like silica containing water as the gel-like silica described above (G2 method). Such gel-like silica as described above can be obtained by controlling a molar ratio of water to silica to a specific range. As described above, the molar ratio of water to silica may also be an important requirement for producing a modified aluminosilicate such as the aluminotitanosilicates which will be described below in the present invention.

The aforementioned Z method will be described in more detail and can be exemplified as a method for contacting the aforementioned sol-like silica containing water, zeolite, with preferably the aforementioned organic structure regulator and the aforementioned crystals (B), preferably stirring and mixing them to prepare a sol at a temperature from 50°C to 100°C, then adjusting a molar ratio of water to silica (HMR-Z) within a range of 0.1 to 8.0 by, for example, evaporating the water, followed by reacting them in a range of 130°C to 700°C (so-called hydrothermal synthesis method). The aforementioned method is a method using sol-like silica, and is thereby expected to be advantageous in terms of reactivity as well as flowability, which is important from an industrial standpoint.

The G2 method described above will be described in more detail. The gel-like silica may generate solids in the course of preparation. Even if such solids are generated, the method for using the crystals (A) described above tends to cause no major problems, however, when using the crystals (B) described above, the performance as the catalyst, as described below, may be lowered. Moreover, in view of industrial processes, the generation of such solids may be disadvantageous from the viewpoint of stable production, because it is thought to raise a risk of interfering with stable and quantitative transfer of the contents and lowering reactivity in the subsequent step due to their solid forms. Therefore, in the G2 method, it is essential to use gel-like silica such as including a solid form, which preferably reduces generation of solids and does not interfere with reactivity in the subsequent step. In order to prepare such suitable gel-like silica, the molar ratio of water to silica (HMR-G2) of the above gel-like silica is preferably adjusted within a range of 3.6 to 6.0. Other preferred conditions are the same as those described above for the first step. The HMR-G2 being within the range as described above has a likelihood of being advantageous in providing a desired alumino(titano)silicate, such as being excellent in reactivity even if solids are generated, and allowing a reaction to sufficiently proceed in the subsequent step.

Preferred examples of the aforementioned crystals (B) can include an aluminosilicate having the highest peak in the region of 20° or more and less than 22.5° in the region of 17.5 to 35° as measured by XRD.

<Method for producing modified aluminosilicate using aluminosilicate (A-1)>

One embodiment of the present invention is a method for producing a modified aluminosilicate, comprising a second step of treating an aluminosilicate (A-1) with an acid, a third step of subjecting the treated product obtained in the second step to primary calcination at 550°C to 850°C, and a fourth step of contacting the calcined product obtained in the third step and liquid containing one or more elements selected from the group consisting of elements in Group 4 and Group 5 of the Periodic Table, followed by carrying out drying and secondary calcination.

Incidentally, each term of the second step to fourth step and the modified aluminosilicate, in the present embodiment is the same as the each term described in the case of using the aluminosilicate (A-1) instead of the aluminosilicate (A-1') in the items of the aforementioned

<Production method of modified aluminosilicate by using aluminosilicate (A-1')>. Specific production methods and preferred conditions are also the same as those when the aluminosilicate (A-1') above is used.

The modified aluminosilicate obtained by the production method of the present invention can be used as a catalyst for production of aromatic polyhydroxide compounds as described below, which is one embodiment of the present invention.

<Method for producing aromatic polyhydroxide compound>

One embodiment of the present invention is a method for producing an aromatic polyhydroxide compound, comprising a step of reacting an aromatic hydroxide with hydroperoxide in the presence of a catalyst for production of aromatic polyhydroxide compounds, containing the aforementioned modified aluminosilicate.

Examples of aromatic hydroxides can include, in addition to the phenols as described below, for example, compounds having a structure in which one hydroxy group is mainly bonded to a phenyl framework, such as hydroxynaphthalene and derivatives thereof, hydroxyanthracene and derivatives thereof, and hydroxyfluorene and derivatives thereof. Phenols are preferred.

Examples of hydroperoxides can include, for example, hydrogen peroxide and compounds in which one hydrogen of hydrogen peroxide is replaced by, for example, an aliphatic group or aromatic hydrocarbon group, or a heteroatom-containing hydrocarbon group, such as butyl hydroperoxide and cumene hydroperoxide. The hydroperoxide is preferably hydrogen peroxide, and in more detail, hydrogen peroxide water is a preferred aspect.

A method for producing an aromatic polyhydroxide will be described below, by using a method for producing aromatic dihydro compounds as an example. For example, when phenols are reacted with hydrogen peroxide in the presence of the modified aluminosilicate of the present invention, aromatic dihydroxide compounds can be produced at high selectivity.

The phenols described above refer to unsubstituted phenols and substituted phenols. Here, examples of the substituted phenols include, for example, alkyl phenols substituted with linear or branched alkyl groups having 1 to 6 carbon atoms, such as a methyl group, ethyl group, isopropyl group, butyl group, or hexyl group, or cycloalkyl group.

Examples of phenols include, for example, phenol, 2-methylphenol, 3-methylphenol, 2,6-dimethylphenol, 2,3,5-trimethylphenol, 2-ethylphenol, 3-isopropylphenol, 2-butylphenol, and 2-cyclohexylphenol, with phenol being preferred among these. Note, however, when the phenols have substituents at both the 2-position and 6-position thereof, a formed product is only a hydroquinone derivative.

Examples of the aromatic dihydroxide compounds that are reaction products include, for example, hydroquinones (substituted or unsubstituted hydroquinones) and catechols (substituted or unsubstituted catechols), and specific examples thereof include, for example, hydroquinone, catechol, 2-methylhydroquinone, 3-methylcatechol, 4-methylcatechol, 3-methylhydroquinone, 1,4-dimethylhydroquinone, 1,4-dimethylcatechol, 3,5-dimethylcatechol, 2,3-dimethylhydroquinone, and 2,3-dimethylcatechol.

The modified aluminosilicate obtained in the present invention is used as a catalyst when producing aromatic dihydroxide compounds. Various methods such as fixed bed, fluidized bed, suspension bed, and shelf-stage fixed bed, may be employed as a method of loading a catalyst, and any one of these methods may be employed. The above catalysts may be also used as they are, or may be molded for use in accordance with methods for loading catalysts. As methods for molding a catalyst, for example, extrusion molding, tableting molding, rolling granulation, and spray granulation are generally employed. When the catalyst is used in the fixed bed method, extrusion molding and tableting molding are preferred. For the suspension bed method, spray granulation is preferred. Drying or calcination may be carried out after spray granulation. An average particle size of catalysts which underwent the spray granulation is preferably in a range of 0.1 $\mu$m to 1,000 $\mu$m and more preferably in a range of 5 $\mu$m to 100 $\mu$m. The average particle size of 0.1 $\mu$m or larger facilitates handling such as catalyst filtration, which is preferred, and the average particle size of 1,000 $\mu$m or smaller provides a catalyst with favorable performance and strength, which is preferred.

The amount of the aforementioned catalyst used is preferably in a range of 0.1 to 30% by mass and more preferably in a range of 0.4 to 20% by mass as an excluded ratio, relative to the total mass of a reaction liquid (total mass of liquid components in the reaction system, not including the mass of fixed components such as a catalyst). The amount of 0.1% by mass or more allows a reaction to complete in a shorter time, improving productivity, which is preferred. The amount of 30% by mass or less results in a small amount of catalyst separated and recovered, which is preferred.

When the modified aluminosilicate of the present invention is used as a catalyst for a production method of aromatic dihydroxide compounds, it can be combined with other components. For example, siloxane compounds as described in Patent Literature 1 and specific alcohol compounds as described in Patent Literature 2, can be included. Such components are preferably used in proportions such that they are each 5 to 90% by mass of the aforementioned reaction liquid. They are more preferably 8 to 90% by mass.

Further, hydrogen peroxide is preferably 0.01 or more and 1 and less in molar ratio with respect to phenols. Concentrations of hydrogen peroxide to be used are also not particularly limited, but an aqueous solution with usual 30% concentration may be used, or hydrogen peroxide water with even higher concentration may be used as it is or diluted with a solvent which is inactive in the reaction system. Examples of solvents used for dilution include, for example, an alcohol and water. Hydrogen peroxide may be added all at once or gradually over time.

A reaction temperature is preferably in a range of 30°C to 130°C and more preferably in a range of 40°C to 100°C. Reactions can proceed at temperatures outside this range, however, the above range is preferred from the viewpoint of improving productivity. Reaction pressure is not particularly limited.

Methods of the aforementioned reaction are not particularly limited, and the reaction may be carried out in a batch manner, semi-batch manner, or continuous manner. When the continuous manner is employed, the reaction may be carried out in a homogeneous mixing tank of suspension-bed type, or in a plug flow manner with a fixed-bed distribution type, or a plurality of reactors may be connected in series and/or in parallel. The number of reactors is preferably from one to four from the standpoint of equipment cost. When a plurality of reactors is used, hydrogen peroxide may be separately added to each thereof.

In order to obtain an aromatic dihydroxide compound from a reaction liquid, a separation liquid after separation of the reaction liquid or the above catalyst, containing a dihydroxide compound, may be subjected to a purification treatment such as removing unreacted components and byproducts. The purification treatment is suitably performed on this separation liquid containing the aromatic dihydroxide compound after separation of the catalyst.

Methods of purification treatments are not particularly limited, and specifically include methods of oil-water separation, extraction, distillation, crystallization, and combinations of these methods. Methods and procedures of the purification treatment are not particularly limited, but for example, the following methods can be used to purify a separation liquid containing the aromatic dihydroxide compound after separation of a reaction liquid and the above catalyst.

When the reaction liquid is separated into two phases of oil phase and water phase, oil and water can be separated. The oil-water separation removes a water phase with low dihydroxide compound content and recovers an oil phase. In this case, the separated water phase may be used to recover an aromatic dihydroxide compound by extraction or distillation, or a portion or all thereof may be used again for a reaction. Moreover, a catalyst separated in the above catalyst separation step or a catalyst which underwent drying treatment can be dispersed in the separated water phase and the mixture can also be fed to a reactor. on the other hand, the oil phase, preferably further undergoes purification treatment by, for example, extraction, distillation, and crystallization.

For extraction, for example, solvents such as 1-butanol, toluene, isopropyl ether, and methyl isobutyl ketone, are used. When the extraction is combined with oil-water separation, the oil-water separation can be carried out efficiently. The extraction solvent is preferably separated and recovered by a distillation column and recycled for use.

Distillation may be carried out on a reaction liquid immediately after catalyst separation or on an oil phase and a water phase after the aforementioned oil-water separation. Furthermore, the extract may be distilled off.

When distilling a reaction liquid immediately after catalyst separation, light boiling components such as water and alcohols are preferably firstly separated. Water and alcohols may be separated in separate distillation columns or in a single distillation column.

After water and alcohols are separated by, for example, the oil-water separation, extraction, and distillation operations described above, phenols may be recovered in the next distillation operation and used again for a reaction. When the recovered phenols contain water that has not been completely separated, the water can be removed by adding isopropyl ether or toluene, by azeotropic distillation.

This azeotropic distillation can also be carried out on liquid after water and alcohols have been separated, before recovery of phenols. The separated water can be used again for a reaction or as wastewater. When the recovered phenols contain impurities such as reaction byproducts other than water, they can also be further separated by distillation operation. In a case in which the impurities are benzoquinones, which are reaction byproducts, they can be supplied to a reactor again together with phenols.

After separation of phenols, a component with a higher boiling point than the aromatic dihydroxide compound can be removed by distillation, and hydroquinones and catechols can be separated by the next distillation operation.

The components with a high boiling point, hydroquinones, and catechols can also be separated in a single distillation operation by extracting the hydroquinones from a middle stage of the distillation column.

The resulting hydroquinones and catechols can be purified by removing impurities through distillation or crystallization, if necessary.

For example, when phenol is reacted with hydrogen peroxide in the presence of the modified aluminosilicate according to the present invention, hydroquinone tends to be formed in high yield. Moreover, hydroquinone tends to be formed at higher selectivity than catechol or benzoquinone. It can be said that for these reasons the modified aluminosilicate according to the present invention is of high industrial value. Aromatic polyhydroxide compounds can also be produced under the same conditions as in the production method of aromatic dihydroxide compounds as described above.

Examples

The present invention will be described in more detail below by way of Examples, however, the present invention is not limited in any way to these Examples.

[XRD measurement method]

X-ray diffraction phenomena of sample crystals were measured by the common methods except for the following conditions.

X-ray diffraction measurement apparatus: Model: MultiFlex manufactured by Rigaku Corporation.
X-ray source: CuK$\alpha$, output: 40kV/40mA, divergence slit: 1°.
Scattering slit: 1°.
Receiving slit: 0.30 mm·2$\theta$: 3 to 50°.

[Example 1]

[Preparation of crystals (A1)]

Crystals (A1) having an MSE framework were prepared by the following method.

First, a container was fed with 15.8 g of an 8 mol/L NaOH aqueous solution, 15.9 g of an 8 mol/L KOH aqueous solution, 31.5 g of a 40% by weight dimethyldipropylammonium hydroxide aqueous solution, and 55 g of colloidal silica (product name: LUDOX (registered trademark) AS-40, manufactured by Sigma-Aldrich Co. LLC), and the mixture was stirred at 60°C to obtain a gel-like substance.

Next, 10.2 g of FAU-type zeolite (product name: HSZ-HUA350, manufactured by TOSOH CORPORATION) was added to the obtained gel-like substance for mixing, and the mixture was then placed in an autoclave and heated at 160°C for 34 hours. The cooled mixture was filtered, washed with water, and vacuum dried at 70°C for 2 hours to obtain crystals (A1). The highest diffraction peak measured by XRD was observed at 23.8°.

[First step: Preparation of aluminosilicate (A-1')]

Next, a container was fed with 15.8 g of an 8 mol/L NaOH aqueous solution, 15.9 g of an 8 mol/L KOH aqueous solution, 31.5 g of a 40% by weight dimethyldipropylammonium hydroxide aqueous solution, 55 g of colloidal silica (product name: LUDOX (registered trademark) AS-40, manufactured by Sigma-Aldrich Co., LLC), and the mixture was stirred at 60°C to obtain a gel-like substance, and 1.47 g of the aforementioned crystals (A1) and 10.2 g of FAU-type zeolite (product name: HSZ-HUA350, manufactured by TOSOH CORPORATION) were added thereto for mixing, and the mixture was placed in an autoclave and heated at 160°C for 68 hours. The cooled mixture was filtered, washed with water, and vacuum dried at 70°C for 2 hours to obtain crystals (1-0).

The highest peak of the above crystals (1-0) in the region of 17.5 to 35° as measured by XRD was observed at 21.6°, and a peak intensity ratio thereof to a peak observed at 26.1° that is the highest peak in the region of 25.0 to 27.0° was 2.07. The above crystals (1-0) were calcined at 550°C for 10 hours to obtain crystals (1-1).

[Second step: Contact with acid, third step: Primary calcination]

After having mixed 3 g of the crystals (1-1) prepared above and 120 g of 60% nitric acid in a container, the mixture was placed in an autoclave and heated at 148°C for 24 hours. The cooled mixture was filtered and washed with water, air dried for 1 day, and then underwent primary calcination at 600°C for 2 hours to obtain crystals (1-2).

[Fourth step: Contact with titanium compound, secondary calcination]

Under a nitrogen atmosphere, a glass container was fed with 2 g of the crystals (1-2) obtained above, to which 100 ml of titanium tetrachloride was added and mixed, and the mixture was heat treated at 120°C for 1 hour. The cooled mixture was filtered, washed with toluene and hexane, and then degassed and dried. Subsequently, secondary calcination was carried out at 600°C for 2 hours to obtain a crystalline porous aluminotitanosilicate, crystals (1-3).

[Example 2]

A container was fed with 1 g of the crystals (1-2) prepared above and they were vacuum degassed at 60°C for 1 hour using an evaporator. 5 g of a 20% titanium trichloride aqueous solution was added thereto, and the mixture was degassed and dried at 70°C for 1 hour. The dried product was subjected to secondary calcination at 600°C for 2 hours to obtain a crystalline porous aluminotitanosilicate (crystals (2-3)).

[Example 3]

Crystals (A3) were obtained in the same manner as in Example 1, except that in the preparation step of the crystals (A1), the mixture was placed in an autoclave and heated at 165°C for 34 hours. The highest diffraction peak measured by XRD was observed at 23.8°.

Crystals (3-0), crystals (3-1), crystals (3-2), and a crystalline porous aluminotitanosilicate (crystals 3-3) were obtained under the same conditions as in Example 2, except that the above crystals (A3) were used instead of the crystals (A1).

The highest peak of the above crystals (3-0) in the region of 17.5 to 35° as measured by XRD was observed at 21.7°, and a peak intensity ratio thereof to a peak observed at 25.8° was 2.04.

[Comparative Example 1]

[Preparation of crystals (A-c1)]

Crystals (A-c1) were obtained under the same conditions as in Example 1, except that the temperature and time upon the preparation of the crystals (A1) in Example 1 were set to 160°C for 68 hours. The highest diffraction peak measured by XRD was observed at 21.6°.

Crystals (C1-0), crystals (C1-1), crystals (C1-2), and a crystalline porous aluminotitanosilicate (crystals C1-3) were obtained under the same conditions as in Example 2, except that the above crystals (A-c1) were used instead of the crystals (A1).

The highest peak of the above crystals (C1-0) in the region of 17.5 to 35° as measured by XRD was observed at 21.6°, and a peak intensity ratio thereof to a peak observed at 26.0° was 1.35.

[Comparative Example 2]

[Preparation of crystals (A-c2)]

Crystals (A-c2) were obtained under the same conditions as in Example 1, except that the temperature and time upon the preparation of the crystals (A1) in Example 1 were set to 160°C for 48 hours. The highest diffraction peak measured by XRD was observed at 22.46°.

Crystals (C2-0), crystals (C2-1), crystals (C2-2), and a crystalline porous aluminotitanosilicate (crystals C2-3) were obtained under the same conditions as in Example 2, except that the above crystals (A-c2) were used instead of the crystals (A1).

The highest peak of the crystals (C2-0) in the region of 17.5 to 35° as measured by XRD was observed at 21.7°, and a peak intensity ratio thereof to a peak observed at 26.2° was 1.42.

[Comparative Example 3]

[Preparation of crystals (A-c3)]

Crystals (A-c3) was obtained under the same conditions as in Example 1, except that the temperature and time upon the preparation of the crystals (A1) in Example 1 were set to 170°C for 34 hours. The highest diffraction peak measured by XRD was observed at 22.3°.

Crystals (C3-0), crystals (C3-1), crystals (C3-2), and a crystalline porous aluminotitanosilicate (crystals C3-3) were obtained under the same conditions as in Example 2, except that the above crystals (A-c3) were used instead of the crystals (A1).

The highest peak of the crystals (C3-0) in the region of 17.5 to 35° as measured by XRD was observed at 21.7° and a peak intensity ratio thereof to a peak observed at 25.8° was 1.66.

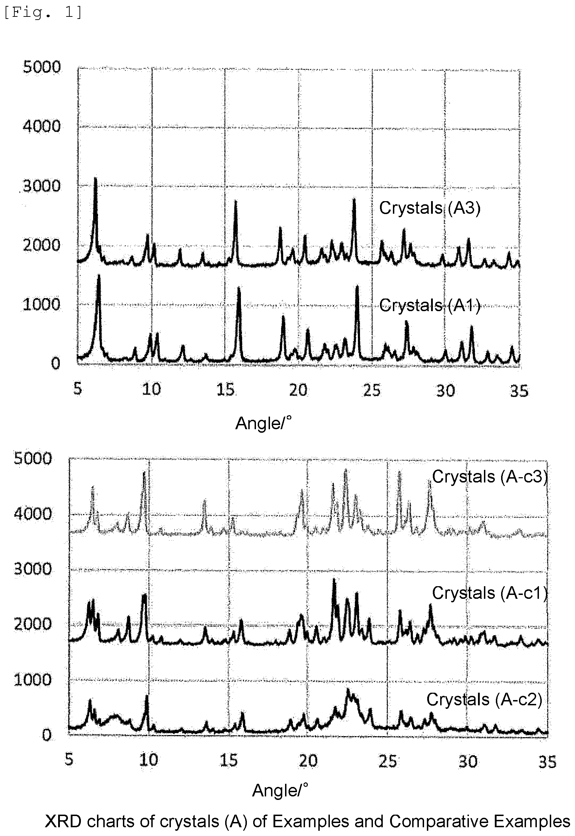

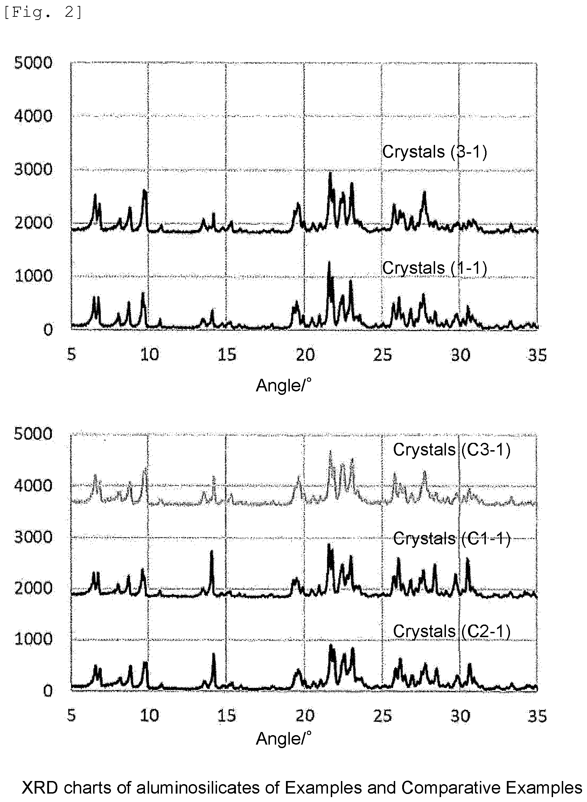

The XRD measurement results of each of the above crystals obtained in Examples 1 to 3 and Comparative Examples 1 to 3 are shown in Figs 1, 2 and 4.

[Performance evaluation as catalyst]

Performance of the crystalline porous aluminotitanosilicates of the above Examples 1 to 3 and Comparative Examples 1 to 3 as catalysts for hydroquinone production was evaluated below. The results are summarized in Table 1. In the table, HQ denotes hydroquinone and CL denotes catechol. The measurement method and the formulae for calculating each value are shown below.

[Measurement method]

To a flask with an internal volume of 50 ml equipped with a cooler, thermometer, feed pump, and magnetic stirrer tip, were added 0.2 g of each catalyst, 4.2 g of phenol, 3.0 g of t-butyl alcohol, and 6.0 g of water, and the mixture was heated to 50°C in a hot water bath while stirring with a stirrer. 0.5 g of 34% hydrogen peroxide was added dropwise from a feed pump over a period of 10 minutes, and the system was maintained for 60 minutes. After cooling the reaction liquid, the catalyst was filtered off, a portion of the reaction liquid was taken out, and the formed product underwent quantitative determination by gas chromatography.

Analytical conditions of gas chromatography are as follows.

Detector: Hydrogen flame ion detector; Column: DB-5 (Agilent J&W), inner diameter 0.25 mm, length 60 m, film thickness 0.25 $\mu$m;
Column temperature: 50°C, held for 10 minutes, temperature rise 10°C/min, up to 280°C;
Inlet; 280°C;
Detector temperature: 280°C;
Carrier gas: Helium;
Flow rate: 80 ml/min.

(Calculation formula)

$$\text{Hydroquinone yield (\%)} = \text{(moles of hydroquinone produced)/(moles of hydrogen peroxide)} \times 100$$

By using the above formula, the hydroquinone yield can also be expressed by the following equation: Hydroquinone yield (%) = (hydrogen peroxide utilization efficiency) × (moles of hydroquinone produced)/[(moles of hydroquinone produced) + (moles of catechol produced)] × 100

$$\text{Hydroquinone/catechol ratio} = \text{(moles of hydroquinone produced)/(moles of catechol produced)}$$

[Table 1]

|  | Crystals (A) | Temperature/°C, Time/hr | Ti source | HQ yield/(%) | HQ/CL ratio |
|---|---|---|---|---|---|
| Example 1 | (A1) | 160°C,34h | TiCl$_4$ | 51.0 | 9.8 |
| Example 2 | (A1) | 160°C,34h | TiCl$_3$ solution | 55.2 | 10.7 |

(continued)

| | Crystals (A) | Temperature/°C, Time/hr | Ti source | HQ yield/(%) | HQ/CL ratio |
|---|---|---|---|---|---|
| Example 3 | (A3) | 165°C, 34h | TiCl$_3$ solution | 49.8 | 10.8 |
| Comparative Example 1 | (A-c1) | 160°C, 68h | TiCl$_3$ solution | 29.5 | 5.8 |
| Comparative Example 2 | (A-c2) | 160°C, 48h | TiCl$_3$ solution | 31.4 | 4.5 |
| Comparative Example 3 | (A-c3) | 170°C, 34h | TiCl$_3$ solution | 34.1 | 7.0 |
| (In the table, the amount of titanium source added denotes that relative to 1 g of dealuminization-UZM-35.) | | | | | |

From Table 1, it is found that the use of the aluminotitanosilicate prepared by using the crystals (A) that meet the requirements of claim 1 of the present invention gave the high hydroquinone yields and high hydroquinone selectivity in both titanium tetrachloride and titanium trichloride aqueous solutions.

The following Examples 4 to 7 and Comparative Examples 4 and 5 are those corresponding to contents of the methods for producing modified aluminosilicates using mainly the aluminosilicates (A-1).

[Example 4]

[Preparation of crystals (A4)]

Crystals (A4) having an MSE framework were prepared by the following method.

First, a container was fed with 7.90 g of an 8 mol/L NaOH aqueous solution, 8.18 g of an 8 mol/L KOH aqueous solution, 20.46 g of a 40% by weight dimethyldipropylammonium hydroxide aqueous solution, 33.82 g of colloidal silica (product name: LUDOX (registered trademark) AS-40, manufactured by Sigma-Aldrich Co. LLC) and the mixture was stirred at 80°C to obtain a sol-like substance. A stirring time was then adjusted to obtain an HMR value of 3.20. At this time, the raw material was solidified.

Next, 6.91 g of FAU-type zeolite was added to the obtained mass (product name: HSZ-HUA350, manufactured by TOSOH CORPORATION) for mixing using a mortar. Thereafter the mixture was then placed in an autoclave and heated at 160°C for 68 hours. The cooled mixture was filtered, washed with water, and vacuum dried at 70°C for 2 hours to obtain crystals (A4). The highest diffraction peak measured by XRD was observed at 21.66°.

[Preparation of aluminosilicate (A-1)]

Next, a container was fed with 7.81 g of an 8 mol/L NaOH aqueous solution, 8.09 g of an 8 mol/L KOH aqueous solution, 24.36 g of a 40% by weight dimethyldipropylammonium hydroxide aqueous solution, and 36.13 g of colloidal silica (product name: LUDOX (registered trademark) AS-40, manufactured by Sigma-Aldrich Co., LLC) and the mixture was stirred at 80°C to obtain a sol-like substance, and 0.79 g of the aforementioned crystals (A1) and 6.78 g of FAU-type zeolite (product name: HSZ-HUA350, manufactured by TOSOH CORPORATION) were added and mixed. A stirring time was then adjusted to obtain an HMR value of 4.00. The autoclave was used to heat the contents at 165°C for 40 hours. The cooled mixture was then filtered, washed with water, and vacuum dried at 70°C for 2 hours to obtain crystals (4-0).

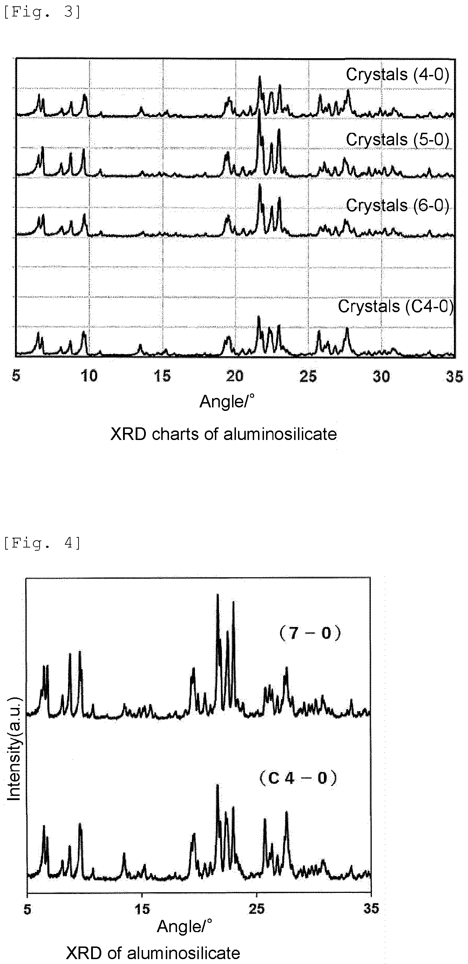

The highest peak of the above crystals (4-0) in the region of 17.5 to 35° as measured by XRD was observed at 21.6°, and a peak intensity ratio thereof to a peak observed at 25.78° that was the highest peak in the region of 25.0 to 27.0° was 1.73. The XRD chart is shown in Fig. 3. The above crystals 4-0 were calcined at 550°C for 10 hours to obtain crystals (4-1).

[Second step: Contact with acid, third step: Primary calcination]

After having fed 2 g of the crystals (4-1) prepared above and 80 g of 65% nitric acid in a container and mixing them, the mixture was placed in an autoclave and heated at 148°C for 24 hours. The cooled mixture was filtered, washed with water, air dried for 1 day, and then underwent primary calcination at 600°C for 2 hours to obtain crystals (4-2).

[Fourth step: Contact with titanium compound, secondary calcination]

A container was fed with 1 g of the crystals (4-2) prepared above and 5 g of a titanium tetrachloride aqueous solution with a Ti concentration of 16%, and the mixture was heated and stirred at 40°C for 1 hour by using an oil bath.

Thereafter, water was added, the mixture was filtered and washed further, and the dried product was subjected to secondary calcination at 650°C for 2 hours to obtain a crystalline porous aluminotitanosilicate (crystals (4-3)).

[Example 5]

[Preparation of crystals (A5)]

Crystals (A5) having an MSE framework were prepared by the following method.

First, a container was fed with 15.60 g of an 8 mol/L NaOH aqueous solution, 16.22 g of an 8 mol/L KOH aqueous solution, 40.60 g of a 40% by weight dimethyldipropylammonium hydroxide aqueous solution, and 67.20 g of colloidal silica (product name: LUDOX (registered trademark) AS-40, manufactured by Sigma-Aldrich Co. LLC) and the mixture was stirred at 80°C to obtain a sol-like substance. A stirring time was then adjusted to obtain an HMR value of 3.50. At this time, the raw material was solidified.

Next, 13.56 g of FAU-type zeolite (product name: HSZ-HUA350, manufactured by TOSOH CORPORATION) was added to the resulting mass and mixed using a mortar. The mixture was then placed in an autoclave and heated at 165°C for 40 hours. The cooled mixture was filtered, washed with water, and vacuum dried at 70°C for 2 hours to obtain crystals (A5). The highest diffraction peak measured by XRD was observed at 21.60°.

[Preparation of aluminosilicate (A-1)]

Next, a container was fed with 7.78 g of an 8 mol/L NaOH aqueous solution, 8.17 g of an 8 mol/L KOH aqueous solution, 24.34 g of a 40% by weight dimethyldipropylammonium hydroxide aqueous solution, and 35.33 g of colloidal silica (product name: LUDOX (registered trademark) AS-40, manufactured by Sigma-Aldrich Co., LLC) and the mixture was stirred at 80°C to obtain a sol-like substance, and 0.79 g of the aforementioned crystals (A2) and 6.78 g of FAU-type zeolite (product name: HSZ-HUA350, manufactured by TOSOH CORPORATION) were added and mixed. A stirring time was then adjusted to obtain an HMR value of 4.80. The mixture was then placed in an autoclave and heated at 160°C for 37 hours. The cooled mixture was then filtered, washed with water, and vacuum dried at 70°C for 2 hours to obtain crystals (5-0).

The highest peak of the above crystals (5-0) in the region of 17.5 to 35° as measured by XRD was observed at 21.6°, and a peak intensity ratio thereof to a peak observed at 26.08° that is the highest peak in the region of 25.0 to 27.0° was 4.30. The XRD chart is shown in Fig. 3. The above crystals (5-0) were calcined at 550°C for 10 hours to obtain crystals (5-1).

[Second step: Contact with acid, third step: Primary calcination]

Crystals (5-2) were obtained in the same manner as in Example 4, except that the crystals (5-1) were used instead of crystals (4-1).

[Third step: Contact with titanium compound, secondary calcination]

A crystalline porous aluminotitanosilicate (crystals (5-3)) was obtained in the same manner as in Example 4, except that the crystals (5-2) were used instead of the crystals (4-2).

[Example 6]

[Preparation of crystals (A6)]

Crystals (A6) having an MSE framework were prepared by the following method.

First, a container was fed with 15.62 g of an 8 mol/L NaOH aqueous solution, 16.22 g of an 8 mol/L KOH aqueous solution, 40.60 g of a 40% by weight dimethyldipropylammonium hydroxide aqueous solution, 67.20 g of colloidal silica (product name: LUDOX (registered trademark) AS-40, manufactured by Sigma-Aldrich Co. LLC) and the mixture was stirred at 80°C to obtain a sol-like substance.

Next, 13.56 g of FAU-type zeolite (product name: HSZ-HUA350, manufactured by TOSOH CORPORATION) was added to the resulting sol-like substance and mixed. A stirring time was then adjusted to obtain an HMR value of 4.00. The mixture was then placed in an autoclave and heated at 165°C for 40 hours. The cooled mixture was filtered, washed with water, and vacuum dried at 70°C for 2 hours to obtain crystals (A6). The highest diffraction peak measured by XRD was observed at 21.66°.

[Preparation of aluminosilicate (A-1)]

Next, a container was fed with 7.81 g of an 8 mol/L NaOH aqueous solution, 8.14 g of an 8 mol/L KOH aqueous solution, 24.39 g of a 40% by weight dimethyldipropylammonium hydroxide aqueous solution, and 36.14 g of colloidal silica (product name: LUDOX (registered trademark) AS-40, manufactured by Sigma-Aldrich Co., LLC) and the mixture was stirred at 80°C to obtain a sol-like substance, and 0.79 g of the aforementioned crystals (A6) and 6.78 g of FAU-type zeolite (product name: HSZ-HUA350, manufactured by TOSOH CORPORATION) were added and mixed. A stirring time was then adjusted to obtain an HMR value of 5.50. The mixture was then placed in an autoclave and heated at 160°C for 50 hours. The cooled mixture was then filtered, washed with water, and vacuum dried at 70°C for 2 hours to obtain crystals (6-0).
The highest peak of the above crystals (6-0) in the region of 17.5 to 35° as measured by XRD was observed at 21.64°, and a peak intensity ratio thereof to a peak observed at 26.14° that was the highest peak in the region of 25.0 to 27.0° was 4.125. The XRD chart is shown in Fig. 3. The above crystals (6-0) were calcined at 550°C for 10 hours to obtain crystals (6-1).

[Second step: Contact with acid, third step: Primary calcination]

Crystals (6-2) were obtained in the same manner as in Example 4, except that the crystals (6-1) were used instead of the crystals (4-1).

[Fourth step: Contact with titanium compound, secondary calcination]

A crystalline porous aluminotitanosilicate (crystals (6-3)) was obtained in the same manner as in Example 4, except that the crystals (6-2) were used instead of the crystals (4-2) .

[Example 7]

[First step: Preparation of aluminosilicate (A-1)]

A container was fed with 7.81 g of an 8 mol/L NaOH aqueous solution, 8.11 g of an 8 mol/L KOH aqueous solution, 24.36 g of a 40% by weight dimethyldipropylammonium hydroxide aqueous solution, 36.08 g of colloidal silica (product name: LUDOX (registered trademark) AS-40, manufactured by Sigma-Aldrich Co. LLC) and the mixture was dehydrated to HMR = 4.0 by stirring at 60°C. The resulting silica compound was in a gel state containing swollen-like solids, which was determined to be usable as was in the next step.
0.79 g of the crystals (A4) and 6.78 g of FAU-type zeolite were added to the solidified raw material mixture (product name: HSZ-HUA350, manufactured by TOSOH CORPORATION). The mixture was then placed in an autoclave and heated at 165°C for 40 hours. The cooled mixture was filtered, washed with water, and vacuum dried at 70°C for 2 hours to obtain crystals (7-0).
The highest peak of the above crystals (7-0) in the region of 17.5 to 35° as measured by XRD was observed at 21.68°, and a peak intensity ratio thereof to a peak observed at 26.18° that was the highest peak in the region of 25.0 to 27.0° was 3.56. The XRD chart is shown in Fig. 4. The above crystals (7-0) were calcined at 550°C for 10 hours to obtain crystals (7-1).

[Second step: Contact with acid, third step: Primary calcination]

After having mixed 2 g of the crystals (7-1) prepared above and 80 g of 65% nitric acid in a container, the mixture was placed in an autoclave and heated at 148°C for 24 hours. The cooled mixture was filtered, washed with water, air dried for 1 day, and then underwent primary calcination at 600°C for 2 hours to obtain crystals (7-2).

[Third step: Contact with titanium compound, secondary calcination]

A container was fed with 0.5 g of the crystals (7-2) prepared above, which were then vacuum degassed at 60°C for 1 hour using an evaporator. 2.5 g of a 20% titanium trichloride aqueous solution was added thereto, and the mixture was degassed and dried at 70°C for 6 hours. The dried product underwent secondary calcination at 600°C for 2 hours to obtain a crystalline porous aluminotitanosilicate (crystals (7-3)).

[Comparative Example 4]

[Preparation of crystals (A-c4)]

Crystals (A-c4) were obtained by the same method as in the preparation of the crystals (A4) in [Example 1].

[Preparation of aluminosilicate]

Next, a container was fed with 7.81 g of an 8 mol/L NaOH aqueous solution, 8.11 g of an 8 mol/L KOH aqueous solution, 20.30 g of a 40% by weight dimethyldipropylammonium hydroxide aqueous solution, 32.68 g of colloidal silica (product name: LUDOX (registered trademark) AS-40, manufactured by Sigma-Aldrich Co. LLC), the mixture was stirred at 80°C, and the stirring was further continued for a longer time than in [Example 4] to dehydrate the mixture to HMR = 3.50. At this time, the mixture was in the state of solidified mass, and therefore pulverization was determined to be necessary in order for the mixture to react with, for example, zeolite in the next step. Therefore, 0.79 g of the aforementioned crystals (A-c4) and 6.78 g of FAU-zeolite were added to the aforementioned mixture, and the mixture was pulverized with a mortar, mixed Adequately, and then charged into an autoclave and heated at 160°C for 55 hours. The cooled mixture was then filtered, washed with water, and vacuum dried at 70°C for 2 hours to obtain crystals (C4-0).
The highest peak of the above crystals (C4-0) in the region of 17.5 to 35° as measured by XRD was observed at 21.58°, and a peak intensity ratio thereof to a peak observed at 25.72° that was the highest peak in the region of 25.0 to 27.0° was 1.54. The XRD charts are shown in Fig. 3 and Fig. 4. The above crystals (C4-0) were calcined at 550°C for 10 hours to obtain crystals (C4-1).

[Contact with acid, primary calcination]

Crystals (C4-2) were obtained in the same manner as in the second step and the third step of Example 4, except that the crystals (C4-1) were used instead of the crystals (4-1).

[Contact with titanium compound, secondary calcination]

Crystals (C4-3) were obtained in the same manner as in the fourth step of Example 4, except that the crystals (C4-2) were used instead of the crystals (4-2).

[Comparative Example 5]

[Contact with titanium compound, secondary calcination]

A container was fed with 0.5 g of the crystals (C4-2) prepared above and the crystals were vacuum degassed at 60°C for 1 hour using an evaporator. 2.5 g of a 20% titanium trichloride aqueous solution was added thereto, and the mixture was degassed and dried at 70°C for 6 hours. The dried product underwent secondary calcination at 600°C for 2 hours to obtain a crystalline porous aluminotitanosilicate (crystals (C5-3)).

[Performance evaluation as catalyst]

Performance of the crystalline aluminotitanosilicates of the above Examples 4 to 7 and Comparative Examples 4 and 5 as catalysts for hydroquinone production was evaluated below. The results are summarized in Table 2. In the table, HQ denotes hydroquinone and CL denotes catechol. The measurement method and the formulae for calculating each value are the same as those in the aforementioned Examples 1 to 3 and Comparative Examples 1 to 3.

[Table 2]

|  | Catalyst-related | | | Performance evaluation | |
|---|---|---|---|---|---|
|  | Ti source | HMR | Peak intensity ratio | HQ yield (%) | HQ/CL ratio |
| Example 4 | TiCl$_4$ solution | 4.00 | 1.73 | 60.32 | 15.0 |
| Example 5 | TiCl$_4$ solution | 4.80 | 4.30 | 48.95 | 18.8 |
| Example 6 | TiCl$_4$ solution | 5.50 | 4.13 | 55.39 | 28.3 |

(continued)

| | Catalyst-related | | | Performance evaluation | |
|---|---|---|---|---|---|
| | Ti source | HMR | Peak intensity ratio | HQ yield (%) | HQ/CL ratio |
| Comparative Example 4 | TiCl$_4$ solution | 3.50 | 1.54 | 42.54 | 13.3 |
| Example 7 | TiCl$_3$ solution | 4.00 | 3.56 | 41.69 | 14.5 |
| Comparative Example 5 | TiCl$_3$ solution | 3.50 | 1.54 | 35.01 | 12.1 |

From Tables 1 and 2, it has been found that the aluminotitanosilicates prepared by the method of producing modified aluminosilicates of the present invention exhibit high hydroquinone yields and high hydroquinone selectivity.

**Claims**

1. A method for producing a modified aluminosilicate, comprising

   a first step of contacting gel-like silica, zeolite, and crystals (A) that is an aluminosilicate with the highest peak ($\alpha$) at 22.5 to 25.0° in a region of 17.5 to 35° as measured by XRD, to obtain an aluminosilicate (A-1'),
   a second step of treating the aluminosilicate (A-1') obtained in the first step with an acid,
   a third step of subjecting the treated product obtained in the second step to primary calcination at 550°C to 850°C, and
   a fourth step of contacting the calcined product obtained in the third step and liquid containing one or more elements selected from the group consisting of elements in Group 4 and Group 5 of the Periodic Table, followed by carrying out drying and secondary calcination.

2. The method for producing a modified aluminosilicate according to claim 1, wherein the crystals (A) in the first step are obtained by a process including a step of contacting gel-like silica with zeolite and treating the same at 155 to 168°C for 30 to 40 hours.

3. An aluminosilicate (A-1), having the highest peak ($\beta$) in a region of 20° or more and less than 22.5° in a region of 17.5 to 35° as measured by XRD, wherein a peak intensity ratio of the peak ($\beta$) to the highest peak ($\gamma$) in a region of 25 to 27° is in a range of 1.7 to 5.0.

4. The aluminosilicate (A-1) according to claim 3, wherein the peak intensity ratio is in a range of 1.7 to 4.0.

5. A catalyst for production of aromatic polyhydroxide compounds, comprising the modified aluminosilicate according to claim 1.

6. A method for producing a modified aluminosilicate, comprising

   a second step of treating the aluminosilicate (A-1) according to claim 3 with an acid,
   a third step of subjecting the treated product obtained in the second step to primary calcination at 550°C to 850°C, and
   a fourth step of contacting the calcined product obtained in the third step and liquid containing one or more elements selected from the group consisting of elements in Group 4 and Group 5 of the Periodic Table, followed by carrying out drying and secondary calcination.

7. A catalyst for production of aromatic polyhydroxide compounds, comprising the modified aluminosilicate according to claim 6.

8. A method for producing an aromatic polyhydroxide compound, comprising a step of reacting an aromatic hydroxide with hydroperoxide in the presence of the catalyst according to claim 5 or claim 7.

[Fig. 1]

XRD charts of crystals (A) of Examples and Comparative Examples

[Fig. 2]

XRD charts of aluminosilicates of Examples and Comparative Examples

[Fig. 3]

XRD charts of aluminosilicate

[Fig. 4]

XRD of aluminosilicate

## INTERNATIONAL SEARCH REPORT

| International application No. |
|---|
| **PCT/JP2022/018562** |

**A. CLASSIFICATION OF SUBJECT MATTER**

*C01B 39/06*(2006.01)i; *C01B 39/48*(2006.01)i; *C07C 39/08*(2006.01)i; *C07C 37/00*(2006.01)i; *B01J 37/08*(2006.01)i;
*B01J 29/89*(2006.01)i
FI:    C01B39/06; B01J29/89 Z; B01J37/08; C01B39/48; C07C37/00; C07C39/08

According to International Patent Classification (IPC) or to both national classification and IPC

**B. FIELDS SEARCHED**

Minimum documentation searched (classification system followed by classification symbols)

C01B39/06; C01B39/48; C07C39/08; C07C37/00; B01J37/08; B01J29/89

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus (JDreamIII); JST7580 (JDreamIII); JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2008-050186 A (YOKOHAMA NATIONAL UNIVERSITY) 06 March 2008 (2008-03-06) manufacturing example 2, example 1, table 2 | 3-4 |
| Y | | 6–8 |
| A | | 1-2, 5 |
| X | JP 2014-043371 A (YOKOHAMA NATIONAL UNIVERSITY) 13 March 2014 (2014-03-13) paragraphs [0021], [0022], example 3, fig. 6 | 3-4 |
| Y | | 6–8 |
| A | | 1-2, 5 |
| X | JP 2018-162183 A (YOKOHAMA NATIONAL UNIVERSITY) 18 October 2018 (2018-10-18) clam 1, comparative example 1-3, paragraphs [0080]-[0084], fig. 2 | 3-4 |
| Y | | 6–8 |
| A | | 1-2, 5 |

☑ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **30 May 2022** | **14 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
|---|
| **PCT/JP2022/018562** |

### C. DOCUMENTS CONSIDERED TO BE RELEVANT

| Category* | Citation of document, with indication, where appropriate, of the relevant passages | Relevant to claim No. |
|---|---|---|
| Y | WO 2019/225549 A1 (MITSUI CHEMICALS INC.) 28 November 2019 (2019-11-28) claims 1-2, 6-7, 15-16, paragraphs [0006]-[0008] | 6–8 |

Form PCT/ISA/210 (second sheet) (January 2015)

**INTERNATIONAL SEARCH REPORT**
Information on patent family members

International application No.

**PCT/JP2022/018562**

| Patent document cited in search report | | Publication date (day/month/year) | Patent family member(s) | | Publication date (day/month/year) |
|---|---|---|---|---|---|
| JP | 2008-050186 A | 06 March 2008 | (Family: none) | | |
| JP | 2014-043371 A | 13 March 2014 | (Family: none) | | |
| JP | 2018-162183 A | 18 October 2018 | (Family: none) | | |
| WO | 2019/225549 A1 | 28 November 2019 | US 2021/0229085 A1 claims 1-2, 6-7, 15-16, paragraphs [0011]-[0014] EP 3798190 A1 CN 112105585 A | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4254009 B
- WO 2015041137 A
- JP 2008050186 A
- JP 2017057126 A
- WO 2019225549 A